# EUROPEAN PATENT SPECIFICATION

(11) **EP 3 016 697 B1**
(45) Date of publication and mention of the grant of the patent: **14.03.2018**
(21) Application number: 14752401.1
(22) Date of filing: 01.07.2014
(51) Int. Cl.: A61M 1/36

(54) **DEVICE FOR FILTERING VENOUS BLOOD**
VORRICHTUNG ZUM FILTERN VON VENÖSEM BLUT
DISPOSITIF POUR FILTRER DU SANG VEINEUX

(30) Priority: 01.07.2013 IT MO20130194
(43) Date of publication of application: 11.05.2016
(73) Proprietor: Eurosets S.r.l., 41036 Medolla (MO) (IT)
(72) Inventor: GHELLI, Nicola, I-40018 S. Pietro in Casale (BO) (IT); PETRALIA, Antonio, I-48022 Lugo (RA) (IT); BALANZONI, Roberto, I-41036 Medolla (MO) (IT); COSTA, Edgardo, I-41036 Medolla (MO) (IT)
(74) Representative: Zoli, Filippo
(86) International application number: PCT/IB2014/062748
(87) International publication number: WO 2015/001474

(56) References cited:
- EP-A1- 0 312 125
- EP-A1- 1 870 118
- EP-A1- 2 394 680
- WO-A1-97/46272
- US-A- 4 737 139

## Description

### Technical Field

The present invention relates to a filtering device of the venous blood.

### Background Art

An extracorporeal circuit of blood circulation adapted to temporarily replace the cardiopulmonary functions is known to be introduced during certain surgical operations.

The extracorporeal blood circuits of known type provide a venous line adapted to convey the blood from the patient (precisely said venous blood) to a filtering device called "venous reservoir", which is in turn connected to a further container, called "cardiotomy reservoir", intended to receive and filter the blood from the surgical field.

The venous reservoir and cardiotomy reservoir are therefore adapted to remove any air bubbles present in the blood and can be integrated into a single device, or separate from one another.

The blood exiting the venous reservoir is then sent, through a pump, to a heat exchanger that regulates its temperature, to an oxygenator which regulates the supply of oxygen to the blood itself, and is finally reintroduced into the patient through an arterial line equipped with a relative filtering device.

The present treatise relates in particular to the filtering device of the venous blood from the patient.

The filtering devices of the venous blood of known type generally comprise a box-shaped casing which defines a filtering chamber in which is arranged an internally hollow filtering element and delimiting a pre-filtering chamber and a post-filtering chamber.

The blood to be filtered is brought into the pre-filtering chamber by means of a supply pipe connected to the venous line which conveys the blood taken from the patient.

For fluid dynamic reasons it is preferable that the blood supply within the pre-filtering chamber takes place from below, so as to facilitate the removal of air (de-bubbling) contained in the venous blood. For this reason, the inlet mouth of the blood to be filtered defined by the supply pipe is generally positioned at the lower part of the pre-filtering chamber.

The filtering devices of known type are generally of two types, which differ in the arrangement of the supply pipe.

A first type envisages that the supply pipe is positioned at the lower portion of the box-shaped casing and therefore is connectable to the venous line, set higher, e.g. by means of a flexible tubular element.

This first type is not very easy to use for health professionals or doctors, since the supply pipe is positioned in an area that is not easily accessible during the operation of the device itself. Moreover, the devices formed this way necessarily envisage that the flexible tubular element has at least a curvilinear length to make the connection with the venous line, with consequent losses of load and the possible formation of stagnant air along it.

A second type envisages on the contrary that the supply pipe is defined at the upper portion of the box-shaped casing and is formed so as to cross the pre-filtering chamber until reaching the proximity of its lower base.

Even this second type has a number of drawbacks.

In fact to ensure the same flow of treated blood compared to a device of the above mentioned first type, it necessarily requires an increase in size of the pre-filtering chamber, so as to offset the volume occupied by the supply pipe. Another drawback is that the blood, when entering the pre-filtering chamber from above, moves in countercurrent with respect to the natural movement of the air bubbles, with the consequent slowing down of the flow of blood and a difficult de-bubbling of the same air bubbles.

Moreover, the known devices of this second type require the use of a diffuser element arranged facing the blood inlet mouth in the pre-filtering chamber defined by the supply pipe, with a consequent increase in the assembly complexity and production costs of the device itself.

US 4737139, WO 97/46272, EP 1870118 and EP 0312125 discloses filtering devices for venous blood of the known type.

### Description of the Invention

The main aim of the present invention is to provide a filtering device of the venous blood which is convenient and easy to use for the operator while being of small size and allowing an efficient de-bubbling of the air bubbles contained in the blood itself.

Within this aim, one object of the present invention is to reduce considerably, compared to the first type of known devices described above, the risk of stagnant air bubbles along the supply pipe.

Yet another object is to provide a filtering device which is structurally simpler than the second type of known devices described above.

Another object of the present invention is to provide a filtering device of the venous blood which allows to overcome the above mentioned drawbacks of the prior art in the ambit of a simple, rational, easy and effective to use as well as low cost solution.

The above mentioned objects are achieved by the present filtering device of the venous blood according to claim 1.

### Brief Description of the Drawings

Other characteristics and advantages of the present invention will become apparent from the description of a preferred, but not exclusive, embodiment of a filtering device of the venous blood, illustrated by way of an indicative, but not limitative, example in the accompanying drawings in which:

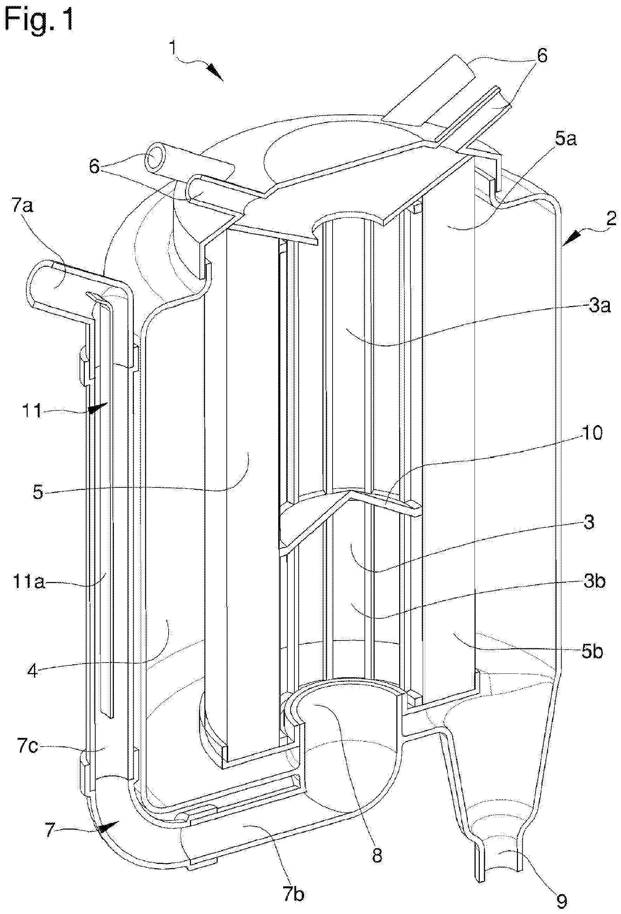

Figure 1 is a first section, in axonometric view, of a filtering device according to the invention;
Figure 2 is a side elevation view of the section of Figure 1;
Figure 3 is a second section of the device of Figure 1;
Figure 4 is a side elevation view of the air fractionation means in a first embodiment;
Figure 5 is a sectional view of the fractionation means of Figure 4 along the track plane V-V;
Figure 6 is a side elevation view of the fractionation means in a second embodiment;
Figure 7 is a sectional view of the fractionation means of Figure 6 along the track plane VII-VII.

### Embodiments of the Invention

With particular reference to these figures, globally indicated by reference number 1 is a filtering device of the venous blood.

The device 1 comprises a box-shaped casing 2, of the rigid type, which defines a filtering chamber 3, 4 within which is arranged at least one internally hollow filtering element 5.

The filtering element 5 therefore delimits a pre-filtering chamber 3, within which the venous blood to be filtered is conveyed, and a post-filtering chamber 4, from which the filtered blood comes out.

More particularly, the filtering element 5 has a substantially tubular conformation, where the pre-filtering chamber 3 and the post-filtering chamber 4 correspond to the volume which is inside and outside the filtering element, respectively. The pre-filtering chamber 3 is therefore delimited laterally only by the filtering element 5, while the post-filtering chamber 4 is delimited laterally on one side by the filtering element 5 and on the other side by the wall of the casing 2.

Suitably, the casing 2 has, in its upper portion, a plurality of attachments 6 communicating with the pre-filtering chamber 3 and connectable to relative blood conveying lines, such as, e.g., a purge line of the arterial filter, a sampling tap, a connection line to a cannula called "intracavitary" cannula directly connected to the patient's heart or an aspirator adapted to draw the blood present in the surgical field.

The device 1 also comprises a supply pipe 7 of the blood to be filtered from the patient within the pre-filtering chamber 3. The supply pipe 7 therefore defines the inlet mouth 8 through which the blood enters the pre-filtering chamber 3 and is associable, on the opposite side, with a conveying line of the venous blood connected to the patient.

According to the invention, the casing 2 is of the monolithic type and defines the supply pipe 7, which is arranged externally to the filtering chamber 3, 4. The supply pipe 7 has a first area 7a, connectable to the conveying line of the venous blood and which is defined at the upper portion of the casing 2, and a second area 7b, associated with the lower portion of the casing 2 and communicating with the pre-filtering chamber 3. The second area 7b therefore defines the inlet mouth 8 facing the pre-filtering chamber 3 to introduce into the latter the venous blood to be filtered.

By the word "monolithic" as used herein it is meant not only a single casing, but also any complex structure composed of several parts joined together and not detachable.

The first area 7a, arranged at the upper portion of the casing 2, allows therefore the easy access by the operator to the supply pipe itself.

The second area 7b, on the other hand, being arranged at the lower portion of the casing 2 allows the introduction from below of the blood to be filtered within the pre-filtering chamber 3, so as to optimize the action of "de-bubbling" of the blood itself.

The supply pipe 7 also has an intermediate area 7c, placed between the first and the second areas 7a and 7b, which extends laterally to the filtering chamber 3, 4. Preferably, as seen in the figures, the intermediate area 7c has at least a length that extends substantially parallel to the filtering element 5.

In the embodiment shown in the illustrations, the portions of the casing 2 that define the areas 7a, 7b and 7c are distinct and integrally associated with each other to define the supply pipe 7.

The words "lower" and "upper" used in this treatise relate to the orientation taken by the device during normal operation.

The pre-filtering chamber 3 then has one or more accesses for the blood to be filtered and the post-filtering chamber 4 has an outlet mouth 9 of the filtered blood, the latter being connectable e.g. to a pump adapted to send the filtered venous blood to an oxygenator.

In the embodiments shown in the illustrations, at least one of the attachments 6 is intended to be connected to a line for the drawing of blood from the surgical field and within the pre-filtering chamber 3 is arranged a separation element 10 adapted to split the pre-filtering chamber 3 into an upper portion 3a, communicating with the attachments 6 and into a lower portion 3b communicating with the supply pipe 7, thus allowing a differentiated filtration for the two flows of blood. In this case the separation element 10 also splits the filtering element 5 into a first part 5a, adapted to filter the blood coming from the attachments 6 and that collects in the upper portion 3a, and into a second part 5b, adapted to filter the blood coming from the supply pipe 7 and that collects in the lower portion 3b. The parts 5a and 5b of the filtering element 5 have a different filtering capacity (i.e. they are adapted to retain impurities of different size).

The separation element 10 preferably has a convex shape upwards. In these embodiments, the device 1 thus serves both as a venous reservoir and as a cardiotomy reservoir.

In an alternative embodiment, not shown in the illustrations, the cardiotomy reservoir is separate and distinct from the device 1, thus the attachments 6 are intended to be connected to at least one of a purge line of the arterial filter, a sampling tap or still an intracavitary cannula directly connected to the patient's heart. In this alternative embodiment, the casing 2 also has a further inlet connector of the blood from the cardiotomy itself and the device 1 is without the separation element 10.

According to the invention, furthermore, the device 1 comprises fractionation means 11 of the air bubbles arranged within the supply pipe 7.

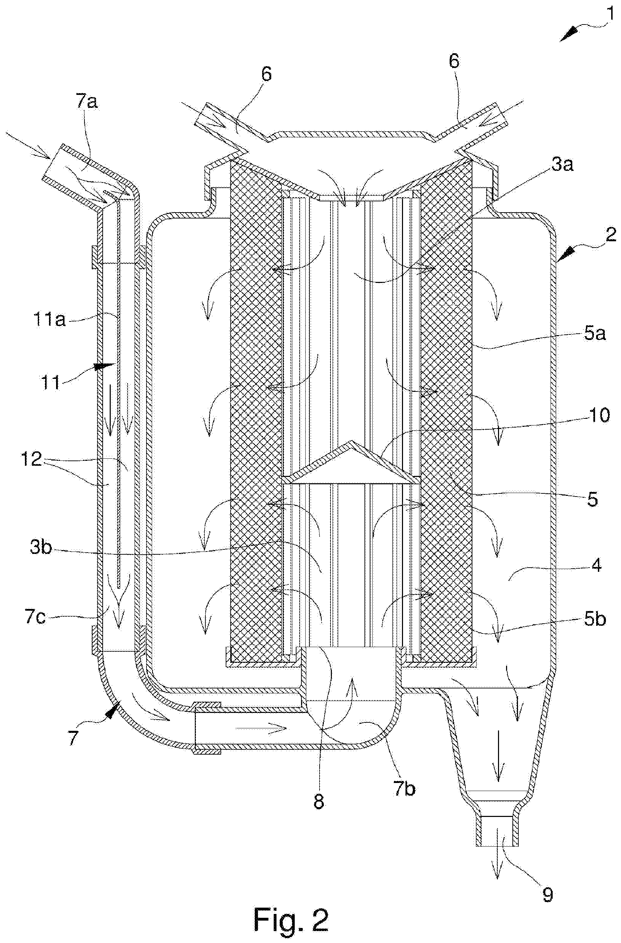

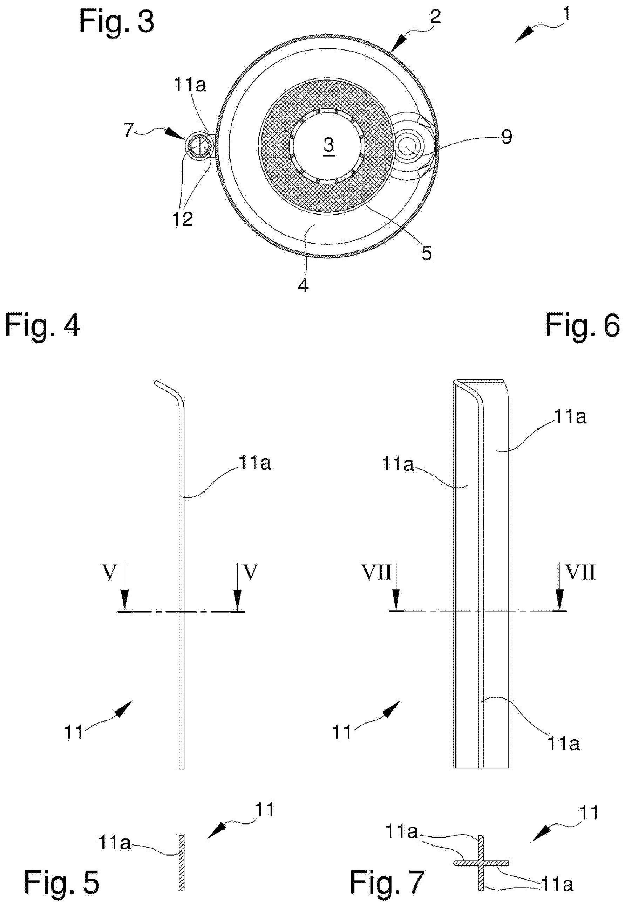

These fractionation means 11 are formed so as to divide the passage section of the supply pipe 7 into two or more channels identified in Figures 2 and 3 with the reference number 12.

More in detail, the fractionation means 11 comprise at least a separation wall 11a of the passage section of the supply pipe 7. The function of the wall 11a is therefore to divide any air bubbles present in the venous blood into bubbles of smaller size, so as to avoid the obstruction of the supply pipe 7 and the consequent reduction in the flow of blood. In fact, since the air bubbles of large size tend to float, moving upwards, they hinder therefore the flow of venous blood, which instead moves downwards, decreasing its flow rate. Following the impact with the fractionation means 11 it happens instead that the air bubbles so crushed, having smaller size, do not have sufficient buoyancy and are therefore easily dragged away downwards by the flow of venous blood without decreasing its flow rate.

In the embodiment shown in the illustrations, the wall 11a is shaped as a lamina and extends along at least a length of the intermediate area 7c of the supply pipe 7.

Preferably, the wall 11a extends substantially along the entire extension of the intermediate area 7c.

It is not ruled out, however, that the wall 11a may be arranged along at least a length of the first and/or second areas 7a and 7b.

Suitably, the wall 11a extends substantially parallel to the longitudinal extension of the supply pipe 7. More in detail, in the embodiments shown in the illustrations, the wall 11a has an extremity area of the wall 11a arranged at the first area 7a of the supply pipe 7 and inclined with respect to the remaining part of the wall itself.

In the first embodiment shown in Figures 4 and 5, the fractionation means comprise only one wall 11a, which therefore splits the supply pipe 7 into two channels 12.

Alternatively, the fractionation means 11 may comprise a plurality of separation walls 11a. More particularly, the walls 11a are arranged radially within the supply pipe 7, i.e. they have one side in common between them and the opposite side arranged at the inner wall of the supply pipe 7.

In the second embodiment shown in Figures 6 and 7, the fractionation means 11 comprise four walls 11a arranged in a cross to define four channels 12.

In an alternative embodiment, not shown in the illustrations, the fractionation means 11 may be the type of an anti foam sponge such as, e.g., an open cell polyurethane foam.

Preferably, the fractionation means 11 comprise at least a fork (not shown in the illustrations) of the supply pipe 7 defined at its first area 7a and adapted to split the flow of venous blood entering the supply pipe itself.

The operation of the present invention is as follows.

To use the device 1, the operator has first to connect the supply pipe 7 and the attachments 6 to the relative conveying lines for the blood to be filtered and the outlet mouth 9 to the devices arranged downstream.

The venous blood to be filtered that enters the supply pipe 7 is split into two or more flows by the fractionation means 11 arranged along the supply pipe itself, as represented by the arrows shown in figure 2.

This implies a division of the flow of blood as well as a fragmentation of any air bubbles of large size present in the venous blood entering the supply pipe itself.

More particularly, the venous blood passes through the separation walls 11a thus entering the channels 12 defined by the same. It follows, therefore, that the air bubbles present in the incoming blood, and in particular those of medium-large size, are fragmented due to the impact with the walls 11a, thereby reducing its size.

The venous blood exiting the channels 12 then reaches the second area 7b of the supply pipe 7 and is conveyed within the pre-filtering chamber 3 passing through the inlet mouth 8 arranged at the lower part of the pre-filtering chamber itself.

The conformation of the supply pipe 7 substantially defines a U-shaped path of the flow of blood, which therefore runs along a first length, which corresponds to the crossing of the first area 7a and of the intermediate area 7c, during which the flow of blood is directed downwards, and a second length, which corresponds to the crossing of the second area 7b and to the entrance in the pre-filtering chamber 3, during which the flow of blood moves upwards.

More particularly, as a result of the crossing of the first area 7a and of the intermediate area 7c, the air bubbles present in the blood are crushed by the fractionation means 11, with a consequent reduction in their buoyancy, and dragged away by the venous blood without this being affected by any slowdown.

Subsequently, or when the venous blood runs along the second area 7b and enters the lower portion 3b of the pre-filtering chamber 3, the flow of venous blood and the air bubbles move in a direction concordant to each other upwards, thus favoring the detachment of the air bubbles (even very small ones).

Air bubbles are therefore grouped below the separation element 10, then they cross the filtering element 5 reaching the post-filtering chamber 4, without incorporating in the blood any longer.

The blood to be filtered entering the lower portion 3b of the pre-filtering chamber 3 then passes through the filtering element 5 and collects in the post-filtering chamber 4.

At the same time within the upper portion 3a of the pre-filtering chamber 3 also enters the blood carried by the other conveying lines connected to the attachments 6.

The blood to be filtered brought by the supply pipe 7 and that conveyed by the attachments 6 is "treated" by the first and by second part 5a and 5b respectively of the filtering element 5 which, as mentioned, are separated from one another by the separation element 10 and have a different filtering capacity.

The filtered blood is then collected in the post-filtering chamber 4 and flows to the outside passing through the outlet mouth 9 as represented by the arrows shown in figure 2 and which identify the path of the blood within the device 1. It has in practice been found how the described invention achieves the proposed objects and in particular the fact is underlined that the particular conformation of the supply pipe of the present invention allows easy access to the operator and obtaining at the same time an effective flow under the fluid dynamic point of view.

Moreover, the presence of the fractionation means allows to avoid the formation of air bubbles of large size and such as to reduce the flow rate of the blood entering the device.

## Claims

1. Filtering device (1) of the venous blood comprising:
- at least a rigid box-shaped casing (2) defining a filtering chamber (3, 4);
- at least an internally hollow filtering element (5) arranged within said filtering chamber (3, 4) to delimit a pre-filtering chamber (3) and a post-filtering chamber (4);
- at least a supply pipe (7) of the blood to be filtered communicating with said pre-filtering chamber (3);
**characterized in that** said box-shaped casing (2) is of the monolithic type and defines said supply pipe (7), the latter being arranged externally to said filtering chamber (3, 4) and comprising a first area (7a) defined at the upper portion of said casing (2) and connectable to a conveying line of the venous blood and a second area (7b) associated with the lower portion of said casing (2) and communicating with said pre-filtering chamber (3), and **in that** it comprises fractionation means (11) of the air bubbles arranged within said supply pipe (7), said fractionation means (11) being formed so as to divide the passage section of said supply pipe (7) into two or more separate channels (12).

2. Device (1) according to claim 1, **characterized in that** said fractionation means (11) comprise at least a separation wall (11a) of the passage section of said supply pipe (7).

3. Device (1) according to claim 2, **characterized in that** said fractionation means (11) comprise a plurality of said separation walls (11a).

4. Device (1) according to claim 3, **characterized in that** said separation walls (11a) are arranged radially within said supply pipe (7).

5. Device (1) according to any of the preceding claims, **characterized in that** said supply pipe (7) has at least one intermediate area (7c) arranged alongside said filtering chamber (3, 4) and placed between said first and second areas (7a, 7b) of the supply pipe itself and **in that** said at least one separation wall (11a) extends along at least a length of said intermediate area (7c).

6. Device (1) according to claim 5, **characterized in that** said at least one separation wall (11a) extends substantially along the entire extension of said intermediate area (7c).

7. Device (1) according to claim 6 or 7, **characterized in that** said first area (7a) of the supply pipe (7) has at least a fork which rejoins at said intermediate area (7c).

8. Device (1) according to any of the preceding claims, **characterized in that** said casing (2) has one or more attachments (6) defined at its upper portion, where at least one of said attachments (6) is intended to be connected to an aspirator adapted to draw the blood present in the surgical field, and **characterized in that** it comprises at least a separation element (10) arranged within said pre-filtering chamber (3) so as to split it into an upper portion (3a) communicating with said attachments (6) and into a lower portion (3b) communicating with said supply pipe (7).

9. Device (1) according to claim 8, **characterized in that** said separation element (10) splits said filtering element (5) into a first part (5a) delimiting the upper portion (3a) of said pre-filtering chamber (3) and into a second part (5b) delimiting the lower portion (3b) of said pre-filtering chamber (3), said first and second parts (5a, 5b) having a different filtering capacity.

## Patentansprüche

1. Filtervorrichtung (1) für venöses Blut, umfassend:
- mindestens ein starres kastenförmiges Gehäuse (2), das eine Filterkammer (3, 4) definiert;
- mindestens ein innen hohles Filterelement (5), das innerhalb der Filterkammer (3, 4) angeordnet ist, um eine Vorfilterkammer (3) und eine Nachfilterkammer (4) abzugrenzen;
- mindestens ein Zufuhrrohr (7) für zu filterndes Blut, das mit der Vorfilterkammer (3) kommuniziert;
**dadurch gekennzeichnet, dass** das kastenförmige Gehäuse (2) vom monolithischen Typ ist und das Zufuhrrohr (7) definiert, wobei das Letztere außerhalb der Filterkammer (3, 4) angeordnet ist und einen ersten Bereich (7a), der im oberen Abschnitt des Gehäuses (2) definiert ist und mit einer Förderleitung des venösen Bluts verbindbar ist, und einen zweiten Bereich (7b), der mit dem unteren Abschnitt des Gehäuses (2) verbunden ist und mit der Vorfilterkammer (3) kommuniziert, umfasst, und dadurch, dass sie Fraktionierungsmittel (11) der Luftblasen umfasst, die in der Zufuhrleitung (7) angeordnet sind, wobei die Fraktionierungsmittel (11) derart gebildet sind, dass sie den Durchgangsquerschnitt des Zufuhrrohrs (7) in zwei oder mehr getrennte Kanäle (12) teilen.

2. Vorrichtung (1) nach Anspruch 1, **dadurch gekennzeichnet, dass** die Fraktionierungsmittel (11) mindestens eine Trennwand (11a) des Durchgangsquerschnitts des Zufuhrrohrs (7) umfassen.

3. Vorrichtung (1) nach Anspruch 2, **dadurch gekennzeichnet, dass** die Fraktionierungsmittel (11) mehrere der Trennwände (11a) umfassen.

4. Vorrichtung (1) nach Anspruch 3, **dadurch gekennzeichnet, dass** die Trennwände (11a) radial innerhalb des Zufuhrrohrs (7) angeordnet sind.

5. Vorrichtung (1) nach Anspruch 3, **dadurch gekennzeichnet, dass** das Zufuhrrohr (7) mindestens einen Zwischenbereich (7c) aufweist, der entlang der Filterkammer (3, 4) angeordnet ist und zwischen dem ersten und zweiten Bereich (7a, 7b) des Zufuhrrohrs selbst platziert ist, und dadurch, dass sich die mindestens eine Trennwand (11a) entlang mindestens einer Länge des Zwischenbereichs (7c) erstreckt.

6. Vorrichtung (1) nach Anspruch 5, **dadurch gekennzeichnet, dass** sich mindestens eine Trennwand (11a) im Wesentlichen entlang der gesamten Ausdehnung des Zwischenbereichs (7c) erstreckt.

7. Vorrichtung (1) nach Anspruch 6 oder 7, **dadurch gekennzeichnet, dass** der erste Bereich (7a) des Zufuhrrohrs (7) mindestens eine Gabelung aufweist, die in dem Zwischenbereich (7c) wieder zusammenläuft.

8. Vorrichtung (1) nach einem der vorhergehenden Ansprüche, **dadurch gekennzeichnet, dass** das Gehäuse (2) eine oder mehrere Ansatzstücke (6) aufweist, die in seinem oberen Abschnitt definiert sind, wobei mindestens eines der Ansatzstücke (6) dazu bestimmt ist, mit einem Aspirator verbunden zu werden, der geeignet ist, das in dem chirurgischen Gebiet vorhandene Blut abzuziehen, und **dadurch gekennzeichnet, dass** sie mindestens ein Trennelement (10) umfasst, das innerhalb der Vorfilterkammer (3) derart angeordnet ist, dass sie in einen oberen Abschnitt (3a), der mit den Ansatzstücken (6) kommuniziert, und in einen unteren Abschnitt (3b), der mit dem Zufuhrrohr (7) kommuniziert, geteilt wird.

9. Vorrichtung (1) nach Anspruch 8, **dadurch gekennzeichnet, dass** das Trennelement (10) das Filterelement (5) in einen ersten Teil (5a), der den oberen Abschnitt (3a) der Vorfilterkammer (3) begrenzt, und in einen zweiten Teil (5b), der den unteren Abschnitt (3b) der Vorfilterkammer (3) begrenzt, teilt, wobei die ersten und zweiten Teile (5a, 5b) eine unterschiedliche Filterkapazität aufweisen.

## Revendications

1. Dispositif de filtration (1) de sang veineux comprenant :
- au moins une enveloppe en forme de boîte rigide (2) définissant une chambre de filtration (3, 4) ;
- au moins un élément de filtration creux à l'intérieur (5) agencé à l'intérieur de ladite chambre de filtration (3, 4) pour délimiter une chambre de pré-filtration (3) et une chambre de post-filtration (4) ;
- au moins un tube d'alimentation (7) du sang à filtrer communiquant avec ladite chambre de pré-filtration (3) ;
**caractérisé en ce que** ladite enveloppe en forme de boîte (2) est du type monolithique et définit ledit tube d'alimentation (7), ce dernier étant agencé à l'extérieur de ladite chambre de filtration (3, 4) et comprenant une première zone (7a) définie au niveau de la portion supérieure de ladite enveloppe (2) et pouvant être reliée à une ligne de transport du sang veineux et une seconde zone (7b) associée à la portion inférieure de ladite enveloppe (2) et communiquant avec ladite chambre de pré-filtration (3), et **en ce que** le dispositif de filtration (1) comprend un moyen de fractionnement (11) des bulles d'air agencé à l'intérieur dudit tube d'alimentation (7), ledit moyen de fractionnement (11) étant formé de façon à diviser la section de passage dudit tube d'alimentation (7) en deux canaux séparés (12) ou plus.

2. Dispositif (1) selon la revendication 1, **caractérisé en ce que** lesdits moyens de fractionnement (11) comprennent au moins une paroi de séparation (11a) de la section de passage dudit tube d'alimentation (7).

3. Dispositif (1) selon la revendication 2, **caractérisé en ce que** lesdits moyens de fractionnement (11) comprennent une pluralité desdites parois de séparation (11a).

4. Dispositif (1) selon la revendication 3, **caractérisé en ce que** lesdites parois de séparation (11a) sont agencées radialement à l'intérieur dudit tube d'alimentation (7).

5. Dispositif (1) selon l'une quelconque des revendications précédentes, **caractérisé en ce que** ledit tube d'alimentation (7) a au moins une zone intermédiaire (7c) agencée le long de ladite chambre de filtration (3, 4) et placée entre lesdites première et deuxième zones (7a, 7b) du tube d'alimentation lui-même et **en ce que** ladite au moins une paroi de séparation (11a) s'étend sur au moins une longueur de ladite zone intermédiaire (7c).

6. Dispositif (1) selon la revendication 5, **caractérisé en ce que** ladite au moins une paroi de séparation (11a) s'étend sensiblement sur toute l'extension de ladite zone intermédiaire (7c).

7. Dispositif (1) selon la revendication 6 ou 7, **caractérisé en ce que** ladite première zone (7a) du tube d'alimentation (7) a au moins une fourche qui se rejoint au niveau de ladite zone intermédiaire (7c).

8. Dispositif (1) selon l'une quelconque des revendications précédentes, **caractérisé en ce que** ladite enveloppe (2) a une ou plusieurs fixations (6) définies au niveau de sa portion supérieure, où au moins une desdites fixations (6) est destinée à être reliée à un aspirateur adapté pour aspirer le sang présent dans le champ chirurgical, et **caractérisé en ce que** le dispositif de filtration (1) comprend au moins un élément de séparation (10) agencé à l'intérieur de ladite chambre de pré-filtration (3) de façon à la diviser en une portion supérieure (3a) communiquant avec lesdites fixations (6) et en une portion inférieure (3b) communiquant avec ledit tube d'alimentation (7).

9. Dispositif (1) selon la revendication 8, **caractérisé en ce que** ledit élément de séparation (10) divise ledit élément de filtration (5) en une première partie (5a) délimitant la portion supérieure (3a) de ladite chambre de pré-filtration (3) et en une deuxième partie (5b) délimitant la portion inférieure (3b) de ladite chambre de pré-filtration (3), lesdites premières et deuxièmes parties (5a, 5b) ayant une capacité de filtration différente.
